# EUROPÄISCHE PATENTSCHRIFT

(11) **EP 3 693 077 B1**
(45) Veröffentlichungstag und Bekanntmachung des Hinweises auf die Patenterteilung: **21.04.2021**
(21) Anmeldenummer: 19155562.2
(22) Anmeldetag: 05.02.2019
(51) Int. Cl.: B01D 53/04, A61M 16/00, A61M 16/10

(54) **ZWEISTUFIGES VERFAHREN DER RÜCKGEWINNUNG VON HALOGENIERTEN KOHLENWASSERSTOFFEN**
TWO-STEP PROCESS FOR THE RECOVERY OF HALOGENATED HYDROCARBONS
PROCÉDÉ EN DEUX ÉTAPES DE RÉCUPÉRATION DES HYDROCARBURES HALOGÉNÉS

(43) Veröffentlichungstag der Anmeldung: 12.08.2020
(73) Patentinhaber: ZeoSys Medical GmbH, 14943 Luckenwalde (DE)
(72) Erfinder: FRIEDRICH, Thomas, 10367 Berlin (DE); EWERS, Christian, 14943 Luckenwalde (DE)
(74) Vertreter: Schulz Junghans Patentanwälte PartGmbB

(56) Entgegenhaltungen:
- EP-A1- 2 926 897
- EP-A2- 0 284 227
- DE-A1-102006 027 127
- DE-A1-102007 048 892
- US-A- 3 332 854
- US-A1- 2016 023 157
- US-A1- 2016 096 793
- US-A1- 2017 056 610

## Beschreibung

Inhalationsanästhetika werden verabreicht, um an einem Patienten eine Narkose einzuleiten oder aufrechtzuerhalten. Die verabreichten Inhalationsanästhetika werden überwiegend über die Atemluft des Patienten wieder an die Umgebung abgegeben werden, weshalb selbige in Operationssälen kontinuierlich abgesaugt und nachteilig für die Umwelt über Dach entsorgt werden. Denn Inhalationsanästhetika aus der Klasse der Flurane bestehen aus halogenierten Kohlenwasserstoffen. Diese sind starke Treibhausgase und ozonschichtschädigend. Die Rückgewinnung von Inhalationsanästhetika aus der Atemluft von Patienten ist daher zum Schutz der Gesundheit von Krankenhauspersonal (in Bereichen ohne Absaugung), aus Umweltschutzgründen und auch aus ökonomischer Sicht unabdingbar.

Es sind bereits Vorrichtungen und Verfahren bekannt, mit denen Inhalationsanästhetika aus der Atemluft von Patienten an Filtermaterialien sowohl adsorbiert als auch wieder desorbiert werden können.

EP2946826 beschreibt eine Filteranlage für ein Gebäude, insbesondere für ein Krankenhaus, die dazu eingerichtet ist, Narkosegase aus dem die Filteranlage durchströmenden Gasgemisch herauszufiltern.

EP2926897 offenbart eine Vorrichtung zur Rückgewinnung von Narkosegasen, insbesondere von halogenierten Kohlenwasserstoffen, die dazu eingerichtet ist, an Filtermaterialien adsorbierte halogenierte Kohlenwasserstoffe mittels Dampf zu desorbieren.

EP2237854 und WO2008/017566 beschreiben ein Verfahren und einen Filter zur Adsorption von halogenierten Kohlenwasserstoffen und deren anschließende Desorption aus dem Filter.

WO2007093640 beschreibt eine Filterpatrone, die zur Adsorption und Desorption von halogenierten Kohlenwasserstoffen eingerichtet ist.

DE 10 2006 027127 offenbart eine Anlage zur Rückgewinnung von halogenierten Kohlenwasserstoffen und Ethern, insbesondere Inhalationsanästhetika, durch Desorption von einem mikroporösen Adsorbens mittels eines Wasserdampfstroms.

US 2016/023157 beschreibt ein Verfahren zur Trennung von Kohlendioxid von einer Gasmischung, wobei die Gasmischung zunächst bei einem ersten Druck an einer Adsorptionseinheit adsorbiert und anschließend bei einem höheren zweiten Druck aus der Adsorptionseinheit ausgeleitet wird.

US 3 332 854 offenbart eine Apparatur zur Rückgewinnung von Lösungsmitteln durch Adsorption an Aktivkohle und anschließende Desorption durch einen Dampfstrom.

EP 0 284 227 beschreibt ein Verfahren zur Rückgewinnung halogenierter Kohlenwasserstoffe aus einem Gasstrom durch Adsorption an einem hydrophoben Molekularsieb-Adsorbens und anschließendes Durchströmen mit einem Spülgas.

DE 10 2007 048892 offenbart einen Narkosegaszwischenspeicher mit einem elektrisch beeinflussbaren Adsorptionsmaterial.

US 2017/056610 offenbart ein System zum Sammeln eines Anästhesiegases, welches das Anästhesiegas in einem Speichersystem adsorbiert.

US 2016/096793 beschreibt ein System und ein Verfahren zur Rückgewinnung von halogenierten Kohlenwasserstoffen aus einem Gasstrom, wobei die Kohlenwasserstoffe an einem Adsorbens mit einer Gitterstruktur adsorbiert und anschließend mit einem Spülgas von dem Adsorbens gelöst werden.

Um rückgewonnene Inhalationsanästhetika erneut zur Narkose von Patienten verwenden zu können, ist es notwendig, die Inhalationsanästhetika zu sterilisieren. Es sind aus dem Stand der Technik noch keine Verfahren und keine Vorrichtungen bekannt, die dazu ausgelegt sind, Inhalationsanästhetika in einem Prozess rückzugewinnen und zu sterilisieren.

### Aufgabe der Erfindung

Aufgabe der Erfindung ist es daher, ein Verfahren und eine Vorrichtung bereitzustellen, mit denen in einem zweistufigen Rückgewinnungsverfahren an einem Filtermaterial adsorbierte Inhalationsanästhetika zunächst sterilisiert und anschließend desorbiert werden können. Diese Aufgabe wird gelöst durch ein Verfahren zur Rückgewinnung von fluorhaltigen Inhalationsanästhetikanach Anspruch 1. Vorteilhafte Ausführungsformen des Verfahrens sind in den Ansprüchen 2 bis 5 dargestellt. Diese Aufgabe wird weiter durch eine Vorrichtung nach Anspruch 6 gelöst. Vorteilhafte Ausführungsformen der Vorrichtung sind in den Ansprüchen 7 und 8 dargestellt.

### Definitionen

Der Begriff *Adsorbens* oder *Sorptionsmittel* bezieht sich im Kontext der vorliegenden Beschreibung auf ein Material, das Gase an seiner Oberfläche adsorbieren kann. *Adsorbens* und *Sorptionsmittel* werden in der vorliegenden Erfindung synonym verwendet.

Der Begriff *Sorbat* bezeichnet das Adsorbens mit den daran adsorbierten Gasen.

Der Begriff *Desorbat* bezeichnet die desorbierten Gase.

Der Begriff *Sorptiv* bezeichnet die zu adsorbierenden Gase.

Unter dem Begriff *halogenierte Kohlenwasserstoffe* sind in der vorliegenden Beschreibung *fluorhaltige Inhalationsanästhetika* zu verstehen.

### Detaillierte Beschreibung der Erfindung

Ein erster Aspekt der Erfindung betrifft ein Verfahren zur Rückgewinnung von halogenierten Kohlenwasserstoffen. In diesem Verfahren wird in einem Desorptionsschritt in aus dem Stand der Technik vorbekannter Weise ein adsorbierte halogenierte Kohlenwasserstoffe umfassendes Adsorbens von einem im Wesentlichen aus trockenem Wasserdampf bestehenden Volumenstrom bei erhöhter Temperatur durchströmt. Dadurch werden die adsorbierten halogenierten Kohlenwasserstoffe vom Adsorbens desorbiert und in den Volumenstrom aufgenommen, wodurch ein halogenierte Kohlenwasserstoffe und Wasserdampf enthaltender sekundärer Volumenstrom entsteht. Dieser sekundäre Volumenstrom wird durch Kühlung in ein halogenierte Kohlenwasserstoffe und Wasser enthaltendes Kondensat überführt. Die Temperatur liegt bei diesem Schritt bei 100°C oder darüber. Verschiedene Verfahrensvarianten sehen eine Durchführung dieses Schrittes bei einer Temperatur von 100 bis 150°C vor, insbesondere bei einer Temperatur von 120°C bis 150°C.

Erfindungsgemäß geht dem Desorptionsschritt ein Sterilisationsschritt voran. Dabei wird das Sorbat vor dem Durchströmen mit Wasserdampf einer heißen Wasserdampfatmosphäre ausgesetzt. Die Erfinder haben die Parameter dieses Schrittes ermittelt, welche notwendig und hinreichend sind, aus potentiell mit pathogenen Organismen kontaminierten Sorbaten wiedergewonnene Wirkstoffe so zu behandeln, dass sie den Vorschriften der Europäischen Arzneimittelbehörden und analoger Vorschriften anderer Länder entsprechen.

Erfindungsgemäß wird während des Sterilisationsschrittes das die adsorbierten halogenierten Kohlenwasserstoffe umfassende Adsorbens für 10 bis 60 min, bei einer Temperatur von 121 bis 150°C, und bei einem Druck von 0,15 MPa bis 0,4 MPa, insbesondere bei einem Druck von 0,15 bis 0,3 MPa in Kontakt mit trockenem Wasserdampf gebracht.

Der Sterilisationsschritt und der Desorptionsschritt werden in einem Desorptionsbehälter in derselben Anlage direkt aufeinanderfolgend durchgeführt,

Der Desorptionsbehälter weist einen Dampfeingang und einen Dampfausgang auf. Das Adsorbens wird zwischen Dampfeingang und Dampfausgang so in dem Desorptionsbehälter angeordnet, dass in den Desorptionsbehälter durch den Dampfeingang eintretender Dampf das Adsorbens durchströmen muss, bevor es den Desorptionsbehälter durch den Dampfausgang verlässt,

Der Desorptionsbehälter weist ein Ventil auf, welches dem Dampfausgang nachgeschaltet ist, wobei das Ventil während des Sterilisationsschrittes geschlossen ist und während des Desorptionsschrittes geöffnet ist.

Die in dem Desorptionsbehälter herrschende Temperatur wird durch einen Temperatursensor gemessen, der sich unterhalb eines in den Desorptionsbehälter eingebrachten Adsorbentienbehälters befindet.

Erfindungsgemäß wurde das die adsorbierten fluorhaltigen Inhalationsanästhetika umfassende Adsorbens durch Filterung von Atemluft aus der Behandlung von Patienten erhalten.

Gemäß einer weiteren Ausführungsform des Verfahrens wird der Sterilisationsschritt für 20 bis 40 min, insbesondere für ungefähr 30 min bei einer Temperatur von 135 bis 145°C, und bei einem Druck von 0,24 bis 0,26 MPa durchgeführt.

Gemäß einer weiteren Ausführungsform des Verfahrens befindet sich der Temperatursensor zwischen dem Dampfausgang und dem in den Desorptionsbehälter eingebrachten Adsorbentienbehälter, insbesondere unmittelbar unterhalb des in den Desorptionsbehälter eingebrachten Adsorbentienbehälters.

Gemäß einer weiteren Ausführungsform des Verfahrens umfasst der Desorptionsbehälter zwei übereinander angeordnete Adsorbentienbehälter und die Adsorbentienbehälter können durch zwei jeweils über den Adsorbentienbehältern angeordnete Dampfeingänge mit Dampf beaufschlagt werden.

Gemäß einer weiteren Ausführungsform des Verfahrens weist der Adsorbentienbehälter einen Boden und/oder einen Deckel auf, wobei der Boden und/oder der Deckel ein gasdurchlässiges Filtergewebe umfassen oder daraus bestehen.

In einer besonderen Ausführungsform wird das die adsorbierten halogenierten Kohlenwasserstoffe umfassende Adsorbens für 20 bis 40 min, insbesondere für ungefähr 30 min, bei einer Temperatur von 135 bis 145°C, und einem Druck von 0,24 bis 0,26 MPa in Kontakt mit trockenem Wasserdampf gebracht.

Durch die Kombination der Parameter Sterilisationszeit, -temperatur und -druck werden Zustände geschaffen, bei denen eine vollständige Inaktivierung aller im Sorbat befindlichen Pathogene gewährleistet wird.

Gleichzeitig sind die oben aufgeführten Parameter Sterilisationszeit, -temperatur und -druck so gewählt, dass die adsorbierten halogenierten Kohlenwasserstoffe unter den herrschenden Bedingungen nicht zersetzt werden oder chemische Reaktionen eingehen, sodass die sterilisierten halogenierten Kohlenwasserstoffe nach der Desorption erneut verwendet werden können.

Während des Sterilisationsschrittes wird das Adsorbens nicht von Wasserdampf durchströmt. Das Adsorbens wird einmal, beim Einleiten des Wasserdampfes, vom Wasserdampf durchströmt und bleibt dann in einer Dampfatmosphäre bei Überdruck. Erst nach Abschließen des Sterilisationsschrittes wird das Adsorbens zur Desorption der halogenierten Kohlenwasserstoffe vom Wasserdampf durchströmt.

Ein weiterer Aspekt der Erfindung ist, dass der Sterilisationsschritt und der Desorptionsschritt in derselben Anlage zeitlich direkt aufeinanderfolgend durchgeführt werden.

Zur Desorption wird der Wasserdampf durch das Sorbat geleitet, sodass eine stetige Wasserdampfströmung durch das Sorbat eine Aufnahme der halogenierten Kohlenwasserstoffe in den Wasserdampf und somit deren Abtransport ermöglicht. Es bildet sich ein Gemisch aus Wasserdampf und halogenierten Kohlenwasserstoffen. Dieses Gemisch wird von den mitgeführten Verunreinigungen befreit und auf eine Temperatur von unter 30 Grad gebracht. Es bildet sich ein zweiphasiges Flüssigkeitsgemisch, das Kondensat. Aus diesem Kondensat werden die halogenierten Kohlenwasserstoffe abgetrennt und weiter verarbeitet. Das Wasser kann in den Prozess zurückgeführt werden.

Nach Desorption der halogenierten Kohlenwasserstoffe wird das Sorptionsmittel gekühlt. Das nun sorptivfreie Sorptionsmittel kann für weitere Ad-/Desorptionszyklen bereitgestellt werden.

Das Adsorbens, an dem die halogenierten Kohlenwasserstoffe adsorbiert werden, ist Aktivkohle, insbesondere hydrophobe Aktivkohle, und/oder Zeolith, insbesondere hydrophober Zeolith, insbesondere modifizierter hydrophober Zeolith. Das Adsorbens ist porös und weist Poren im Bereich von Mikrometern und/oder Nanometern auf. Es kann auch ein Adsorbentiengemisch verwendet werden.

In bestimmten Ausführungsformen der Erfindung ist das Adsorbens vor der Adsorption bzw. nach der Durchführung des gesamten Verfahrenszyklus und vor Einsatz zur Adsorption von Narkosemitteln durch einen Wassergehalt von ≤5% (w/w), insbesondere durch einen Wassergehalt von ≤2% (w/w) charakterisiert. Dieser Wasseranteil kann sich im Laufe des Verfahrens ändern.

Adsorbentien können sowohl hydrophil als auch hydrophob sein. Für das vorliegende Verfahren werden hydrophobe Adsorbentien verwendet. Auch hydrophobe Adsorbentien können große Mengen Wasser aufnehmen. Eine Adsorption ist nur an den nicht durch Wasser besetzten Stellen des Adsorbens möglich. So soll das erfindungsgemäß verwendete Adsorbens einen möglichst niedrigen Wassergehalt aufweisen, um möglichst viel halogenierte Kohlenwasserstoffe aufnehmen zu können. Nach der Desorption der halogenierten Kohlenwasserstoffe kann das Adsorbens einen Wassergehalt von über 30% (w/w) aufweisen. Nach Entfernung eines Großteils des Wassers kann das Adsorbens wiederverwendet werden.

Der im Desorptionsschritt verwendete Wasserdampf enthält im Wesentlichen kein flüssiges Wasser.

Man unterscheidet zwischen Nassdampf und Heißdampf. Heißdampf ist Dampf, der eine Temperatur oberhalb der Siedetemperatur des Wassers bei dem gegebenen Druck aufweist. Heißdampf enthält somit kein flüssiges Wasser mehr. Beim Nassdampf gibt es immer einen Anteil an Wassertröpfchen im Dampf.

Der verwendete Wasserdampf ist in einer besonderen Ausführungsform Reinstdampf. Reinstdampf wird durch Verdampfen von vollentsalztem Wasser erhalten.

Die halogenierten Kohlenwasserstoffe umfassen fluorhaltige Inhalationsanästhetika, insbesondere Sevofluran, Isofluran, Enfluran, Halothan, Desfluran oder Gemische daraus.

Das die halogenierten Kohlenwasserstoffe umfassende Adsorbens wurde durch Filterung von Atemluft aus der Behandlung von Patienten erhalten, die mit halogenierten Kohlenwasserstoffen narkotisiert wurden.

Ein weiterer Aspekt der Erfindung ist eine Vorrichtung zur Durchführung des erfindungsgemäßen Verfahrens, umfassend einen druckstabilen Desorptionsbehälter, einen zum Einlass von Wasserdampf in den Desorptionsbehälter eingerichteten Dampfeingang, einen Dampferzeuger, der zur Erzeugung von Wasserdampf eingerichtet ist, wobei der Dampferzeuger mit einem Dampfeingang in fluidischer Kommunikation steht, einen zum Abführen von Wasserdampf aus dem Desorptionsbehälter eingerichteten Dampfausgang mit einer in Richtung des Dampfaustritts hinter dem Dampfausgang angeordneten Ausleitung; ein Ventil, durch welches die Ausleitung verschließbar ist, einen Raum, der zur Aufnahme eines Schüttguts ausgebildet ist, wobei das Schüttgut ein fluorhaltige Inhalationsanästhetika umfassendes Adsorbens ist, wobei der Desorptionsbehälter einen zur Aufnahme des Schüttguts vorgesehenen Adsorbentienbehälter, welcher den Raum zur Aufnahme des Schüttguts ausbildet, und einen Temperatursensor, welcher auf der dem Dampfausgang zugewandten Seite des Adsorbentienbehälters angeordnet ist, umfasst, und einen mit dem Desorptionsbehälter verbundenen Kondensatbereich, der dazu eingerichtet ist, einen fluorhaltige Inhalationsanästhetika enthaltenden sekundären Volumenstrom zu kühlen, der in einem Desorptionsschritt durch Durchströmen des fluorhaltige Inhalationsanästhetika umfassenden Adsorbens mit dem Wasserdampf entsteht, und aus dem sekundären Volumenstrom ein Kondensat zu erzeugen.

Gemäß einer Ausführungsform der Vorrichtung ist der Temperatursensor mit einer Steuereinrichtung verbunden, welche dazu ausgebildet ist, das Ventil nach Erreichen einer Zieltemperatur und/oder nach Ablauf einer vorgewählten Zeitspanne von 10 bis 60 min, während derer die Zieltemperatur gehalten wird, zu öffnen.

Erfindungsgemäß ist der Desorptionsbehälter bis 0,4 MPa druckstabil.

Gemäß einer weiteren Ausführungsform der Vorrichtung ist der Desorptionsbehälter zur Aufnahme zweier übereinander angeordneter Adsorbentienbehälter eingerichtet, und mit zwei jeweils oberhalb der Adsorbentienbehälter angeordneten, Dampfeingängen ausgestaltet.

Ein weiterer Aspekt der Erfindung ist, dass der Sterilisationsschritt und der Desorptionsschritt in einem bis 0,4 MPa druckstabilen Desorptionsbehälter (100) durchgeführt werden. Der Desorptionsbehälter weist mindestens einen Dampfeingang (110) und mindestens einen Dampfausgang (120) auf. Über den Dampfeingang (110) wird Dampf in den Desorptionsbehälter (100) eingespeist und verlässt den Desorptionsbehälter (100) über den Dampfausgang (120). Das die adsorbierten halogenierten Kohlenwasserstoffe umfassende Adsorbens ist zwischen Dampfeingang (110) und Dampfausgang (120) so in dem Desorptionsbehälter (100) angeordnet, dass in den Desorptionsbehälter (100) durch den Dampfeingang (110) eintretender Dampf das Adsorbens durchströmen muss, bevor es den Desorptionsbehälter (100) durch den Dampfausgang (120) verlässt. Jeder Punkt des die adsorbierten halogenierten Kohlenwasserstoffe umfassenden Adsorbens kommt so in Berührung mit dem Dampf. Beim Durchströmen des Adsorbens mit Wasserdampf bildet sich ein Temperaturgradient vom Dampfeingang (110) zum Dampfausgang (120) aus. Der kälteste Punkt in dem Desorptionsbehälter befindet sich somit am Dampfausgang (120). An dieser Stelle ist ein Temperatursensor (150) angebracht.

Der Desorptionsbehälter (100) weist ein Ventil (140) auf, welches dem Dampfausgang (120) nachgeschaltet ist, wobei das Ventil (140) während des Sterilisationsschrittes geschlossen ist und während des Desorptionsschrittes geöffnet ist. Somit kann sich während des Sterilisationsschrittes der erforderliche Druck im Desorptionsbehälter (100) einstellen. Während des Desorptionsschrittes ist das Ventil (140) geöffnet, sodass der sekundäre Volumenstrom den Desorptionsbehälter (100) über den Dampfausgang (120) verlassen kann.

Das die halogenierten Kohlenwasserstoffe umfassende Adsorbens kann im Desorptionsbehälter (100) in einem dem Desorptionsbehälter (100) entnehmbaren Adsorbentienbehälter (200) aufgenommen sein.

Der Desorptionsbehälter (100) weist eine umlaufende Wand (170) und einen mit der umlaufenden Wand (170) abschließenden Boden (130) auf, wobei der Boden (130) ein gewölbter Boden, insbesondere ein Klöpperboden nach DIN 28011 ist. Der Boden (130) bildet durch seine Wölbung einen Hohlraum unterhalb des in den Desorptionsbehälter (100) eingebrachten Adsorbentienbehälters (200) aus, und ist zur Aufnahme eines Kondensats eingerichtet. Das Kondensat umfasst kondensierte Gase. Das Kondensat kann sowohl Wasserdampf als auch die halogenierten Kohlenwasserstoffe umfassen. Der Boden ist nicht zwingend als Klöpperboden ausgebildet, aber für das erfindungsgemäße Verfahren von Vorteil, da somit das Desorbat-Wasser-Gemisch ohne Restverbleib über das Ausgangsventil weitergeleitet werden kann.

Der Boden (130) ist so gestaltet, dass er die maximal mögliche Menge an Kondensat unter Betriebsbedingungen aufnehmen kann. Die Menge aufgenommenen Kondensats hängt von der Temperaturdifferenz zum Desorptionsbehälter ab. In bestimmten Ausführungsformen ist vorgesehen, den Boden des Behälters noch vorzuheizen, um die Menge an Kondensat möglichst niedrig zu halten. Eine Restmenge an Kondensat ist in den meisten Betriebszuständen schwer zu vermeiden, da eine einmal gebildete Kondensatmenge durch die kleine Wärmeübertragungsfläche nicht mehr durch den nachfolgenden Dampf vollständig in die Gasphase überführt werden kann.

Die in dem Desorptionsbehälter (100) herrschende Temperatur wird durch einen Temperatursensor (150) gemessen und gesteuert. Der Temperatursensor (150) befindet sich unterhalb des in den Desorptionsbehälter (100) eingebrachten Adsorbentienbehälters (200), insbesondere zwischen dem Dampfausgang (120) und dem in den Desorptionsbehälter (100) eingebrachten Adsorbentienbehälter (200), insbesondere unmittelbar unterhalb des in den Desorptionsbehälter (100) eingebrachten Adsorbentienbehälters (200).

Der Temperatursensor (150) befindet sich unmittelbar an der Unterseite des Adsorbentienbehälters (200). Er ist so angeordnet, dass das Sensorelement sich genau unterhalb des Bodens des Adsorbentienbehälters (200) befindet. Der Temperatursensor (150) misst so immer die Temperatur der Dampfphase und nicht die des Kondensats, welches sich im Bereich des Bodens (130) des Desorptionsbehälters (100) ansammeln kann.

Der Temperatursensor (150) ist dazu eingerichtet, das Verfahren zu steuern. Sobald während des Sterilisationsschrittes eine vorgegebene Sterilisationstemperatur erreicht ist, wird eine eingestellte Sterilisationszeit gemessen. Nach Ablauf der Sterilisationszeit wird der Sterilisationsschritt beendet. Dies kann durch Öffnen des Ventils (140) und somit durch Einleiten des Desorptionsschrittes erfolgen.

In bestimmten Ausführungsformen dient der Temperatursensor (150) der Überwachung und der Steuerung des erfindungsgemäßen Verfahrens, aber nur soweit, dass der Sterilisationsprozess bei Unterschreiten der eingestellten Temperatur neu gestartet wird. Eine Verbindung mit dem Dampferzeuger im Prozess ist nicht gegeben. In diesen Ausführungsformen wird der Prozess selber nur druckseitig über den eingestellten Betriebsdruck gefahren.

In bestimmten andere Ausführungsformen dient der Temperatursensor (150) der Überwachung und der Steuerung des erfindungsgemäßen Verfahrens durch direkte Steuerung des Dampferzeugers und ggf. der angeschlossenen Ventile.

In bestimmten Ausführungsformen umfasst der Desorptionsbehälter (100) zwei übereinander angeordnete Adsorbentienbehälter (200a, 200b). Die Adsorbentienbehälter (200a, 200b) werden durch zwei jeweils über oder vor den Adsorbentienbehältern angeordnete Dampfeingänge (110a, 110b) mit Dampf beaufschlagt. Durch die Aufnahme von zwei Adsorbentienbehältern (200a, 200b) mit jeweils einem eigenen Dampfeingang (110a, 110b) verringert sich der Temperaturgradient innerhalb des Adsorbens. Eine Sterilisation ist somit auch bei einer geringeren Dampftemperatur, im Vergleich zu einer Vorrichtung mit nur einem Dampfeingang (110), an jeder Stelle des Adsorbens erfolgreich.

Der Adsorbentienbehälter (200) umfasst eine umlaufende Wand (220), mit der umlaufenden Wand (220) abschließend einen Boden (230) und/oder einen Deckel (240). In bestimmten Ausführungsformen umfasst der Boden (230) und/oder der Deckel (240) ein gasdurchlässiges Filtergewebe (250), oder besteht daraus. Das Filtergewebe (250) weist Poren im Bereich von 10-100 µm auf, insbesondere in einem Bereich von 20-50 µm, insbesondere von 40 µm.

Der/die Adsorbentienbehälter (200) können in den Desorptionsbehälter (100) eingeführt werden.

Die Wand des Adsorbentienbehälters (220) kontaktiert die Wand des Desorptionsbehälters (170) nicht, wodurch sich ein Zwischenraum zwischen Desorptionsbehälterwand (170) und Adsorbentienbehälterwand (220) ausbildet. Zur Vermeidung eines Dampfstroms im Zwischenraum zwischen Adsorbentienbehälter (200) und Desorptionsbehälter (100) ist der Deckel des Adsorbentienbehälters (240) mit einer Dichtung (210) versehen, so dass der Dampf den Adsorbentienbehälter (200) passieren muss. Diese Dichtung (210) ist in bestimmten Ausführungsformen als aufblasbare Profildichtung ausgeführt.

Ein weiterer Aspekt des Verfahrens ist die Rückgewinnung der halogenierten Kohlenwasserstoffe im Rückgewinnungsschritt. Hierzu wird der sekundäre Volumenstrom, der den Desorptionsbehälter über den Dampfausgang verlässt, über eine Sammelleitung mittels eines spülbaren Vorfilters und eines spülbaren Nachfilters von den mitgeführten Verunreinigungen befreit und durch nacheinander geschaltete Kühler, insbesondere drei solcher nachgeschalteten Kühler, auf eine Temperatur von unter 30 Grad gebracht. Nach dem letzten Kühler wird das so gebildete Kondensat-Wasser-Gemisch in einen Kondensatauffangbehälter überführt. Es bildet sich ein zweiphasiges Flüssigkeitsgemisch. Aus diesem Kondensat werden die halogenierten Kohlenwasserstoffe abgetrennt und weiter verarbeitet. Das Wasser kann in den Verdampfungsvorgang zurückgeführt werden.

Ein weiterer Aspekt der vorliegenden Erfindung umfasst eine Vorrichtung zur Durchführung des dargestellten zweistufigen Verfahrens der Rückgewinnung von halogenierten Kohlenwasserstoffen. Diese Vorrichtung umfasst einen druckstabilen Desorptionsbehälter (100). Dieser Desorptionsbehälter (100) umfasst
- einen zum Einlass von Wasserdampf in den Desorptionsbehälter (100) eingerichteten Dampfeingang (110),
- einen zum Abführen von Wasserdampf aus dem Desorptionsbehälter (100) eingerichteten Dampfausgang (120) mit einer in Richtung des Dampfaustritts hinter dem Dampfausgang (120) angeordneten Ausleitung (190),
- ein Ventil (140), durch welches die Ausleitung (190) verschließbar ist,
- einen Raum zwischen Dampfeingang (110) und Dampfausgang (120), der zur Aufnahme eines Schüttguts, z.B. ein halogenierte Kohlenwasserstoffe umfassendes Adsorbens, ausgebildet ist.

Das Ventil (140) ist druckstabil bis mindestens zu einem Druck von 0,4 MPa verschließbar. Das Ventil (140) ist dazu eingerichtet, den Druck innerhalb des Desorptionsbehälters (100) zu steuern. Ist das Ventil geschlossen, ist es dazu ausgebildet, einen Druckaufbau innerhalb des Desorptionsbehälters (100) zu ermöglichen. Der Dampfausgang (120) ist bei geschlossenem Ventil (140) versperrt. Ist das Ventil (140) geöffnet, kann ein Volumenstrom den Desorptionsbehälter (100) über den Dampfausgang (120) verlassen.

Der Desorptionsbehälter (100) umfasst einen zur Aufnahme des Schüttguts vorgesehenen entnehmbaren Adsorbentienbehälter (200) und einen Temperatursensor (150), welcher auf der dem Dampfausgang (120) zugewandten Seite und damit auf der dem Dampfeingang (110) abgewandten Seite des Adsorbentienbehälters (200) angeordnet ist. Der Temperatursensor (150) ist unmittelbar unterhalb des Adsorbentienbehälters angeordnet.

Der Temperatursensor ist (150) mit einer Steuereinrichtung (160) verbunden, welche dazu ausgebildet ist, das Ventil (140) nach Erreichen einer Zieltemperatur und/oder nach Ablauf einer vorgewählten Zeitspanne von 10 bis 60 min, während derer die Zieltemperatur gehalten wird, zu öffnen. Die Steuereinrichtung (160) kann sich außerhalb des Desorptionsbehälters (100) befinden.

Der Desorptionsbehälter (100) weist einen gewölbten Boden (130), insbesondere einen Klöpperboden nach DIN 28011 auf. Der Boden (130) ist dazu eingerichtet, während des Sterilisationsschrittes kondensierte Anteile aufzunehmen.

Der Desorptionsbehälter (100) ist bis 0,4 MPa druckstabil.

Der Desorptionsbehälter (100) ist zur Aufnahme zweier übereinander angeordneter Adsorbentienbehälter (200a, 200b) eingerichtet, und mit zwei jeweils oberhalb der Adsorbentienbehälter (200a, 200b) angeordneten, Dampfeingängen (110a, 110b) ausgestaltet.

Die Vorrichtung weist einen Dampferzeuger (300) auf, der zur Erzeugung trockenen Wasserdampfes eingerichtet ist, wobei der Dampferzeuger (300) mit mindestens einem Dampfeingang (110) verbunden ist.

Eine dem Dampferzeuger (300) nachgeschaltete Trocknungseinrichtung (310) stellt einen Heißdampfzustand unter den gegebenen Bedingungen her. Die Trocknungseinrichtung (310) kann eine Tröpfchenabscheidekolonne sein.

Der Dampferzeuger weist einen unteren und einen oberen Schaltpunkt zur Füllstandskontrolle auf. Der Wasserstand kann kontrolliert werden, damit der Dampferzeuger nicht trocken läuft bzw. nicht überläuft und somit Dampf in gewünschter Qualität bereitgestellt werden kann.

Es kann vorgesehen sein, dass bei Unterschreiten eines vorbestimmten Füllstands der Dampferzeuger bzw. die einzeln geschalteten Patronen a 5 KW automatisch abgeschaltet wird, um das Trockenlaufen und Durchglühen zu verhindern.

Es ist möglich, dass der Dampferzeuger (300) mit Dampfeingängen (110) mehrerer Desorptionsbehälter (100) verbunden ist. In einer Ausführungsform ist der Dampferzeuger (300) mit vier Desorptionsbehältern (100) verbunden. Die maximale Produktionsleistung Reinstdampf liegt in dieser Ausführungsform bei 60 kg/h, 0,2 MPa (1 Atmosphäre Überdruck). Diese wird im Parallelanfahrbetrieb von vier Desorptionsbehältern (100) voll genutzt. Im weiteren Betrieb liegt diese dann bei annähernd einem Drittel bis der Hälfte davon.

Die Desorptionsbehälter (100) werden in unterschiedlicher Anzahl mit einem Zeitverzug von 1 bis 2 min parallel gefahren.

Der Dampferzeuger (300) ist dazu eingerichtet, aus vollentsalztem Wasser Reinstdampf herzustellen.

Das vollentsalzte Wasser wird durch eine Wasseraufbereitungsvorrichtung bereitgestellt, die dem Dampferzeuger (300) vorgeschaltet ist. Diese umfasst eine Stadtwassereinspeisung (410), eine Enthärtungsanlage (420) und nachfolgend ein Umkehrosmosemodul (430). Das aufbereitete Wasser wird in einem mit dem Dampferzeuger (300) verbundenen Wasserspeicher (440) gespeichert. Zwischen der Stadtwassereinspeisung (410) und der Enthärtungsanlage (420) ist ein Vorfilter (450) eingebaut. Der Vorfilter (450) weist Poren mit einer Größe von 50 µm bis maximal 100 µm auf. Zwischen der Enthärtungsanlage (420) und dem Umkehrosmosemodul (430) ist ein Feinfilter (460) angeordnet. Die Poren des Feinfilters (460) sind 2 µm bis 8 µm groß, wobei die Poren unterschiedlich groß sein können. Die durchschnittliche Größe beträgt in bestimmten Ausführungsformen 5 µm.

Der Desorptionsbehälter (100) ist mit einem Kondensatbereich verbunden, der dazu eingerichtet ist, den sekundären Volumenstrom zu kühlen und daraus ein Kondensat zu erzeugen. Zwischen Desorptionsbehälter (100) und Kondensatbereich ist ein Filter (510) angeordnet. Dieser Filter (510) ist dazu eingerichtet, alle im sekundären Volumenstrom mitgeführten Partikel, die sich beim Durchströmen des Adsorbens davon lösen, herauszufiltern. Dieser Filter (510) ist spülbar. Es ist möglich, mehrere dieser Filter (510) hintereinander anzuordnen.

In einer Ausführungsform sind zwischen Desorptionsbehälter (100) und Kondensatbereich ein spülbarer Vor- (510a) und ein spülbarer Nachfilter (510b) angeordnet, wobei der Vorfilter (510a) dem Nachfilter (510b) vorgeschaltet ist. Der Vorfilter (510a) weist eine Porengröße von 15 µm bis 30 µm, insbesondere von ca. 25 µm auf, der Nachfilter (510b) weist eine Porengröße von 1 µm bis 5 µm, insbesondere von ca. 5 µm auf.

Der Kondensatbereich umfasst einen Vorkühler (520), einen Zwischenkühler (530) sowie einen Nachkühler (540), die nacheinander angeordnet sind. Vor- (520) und Zwischenkühler (530) werden mit Stadtwasser betrieben. Der Nachkühler (540) wird mit gekühltem Stadtwasser betrieben. Zwischen Zwischen- (530) und Nachkühler (540) ist ein Feinfilter (550) angeordnet. Der Feinfilter (550) weist eine Porengröße von 1 µm bis 5 µm, insbesondere von ca. 5 µm auf.

An den Kondensatbereich schließt sich ein Auffangbereich an. Dieser umfasst einen Kondensatauffangbehälter (610), einen Drucklufterzeuger sowie einen Pufferbehälter (630).

Der Kondensatauffangbehälter (610) schließt sich stromabwärts an den Nachkühler (540) an. Dieser Kondensatauffangbehälter (610) ist dazu eingerichtet, das gekühlte Kondensat aufzufangen und in seine Phasen aufzutrennen.

Bei Erreichen einer entsprechenden Füllhöhe werden die halogenierten Kohlenwasserstoffe, die sich auf Grund ihrer Dichte vom Wasser trennen, mittels eines Auslasses in einen Pufferbehälter (630) überführt.

### Figurenbeschreibung

Fig. 1 zeigt einen Desorptionsbehälter (100) mit zwei entnehmbaren Adsorbentienbehältern (200a, 200b).
Fig. 2 zeigt einen Adsorbentienbehälter (200).
Fig. 3 stellt einen schematischen Aufbau der Rückgewinnungsanlage dar.

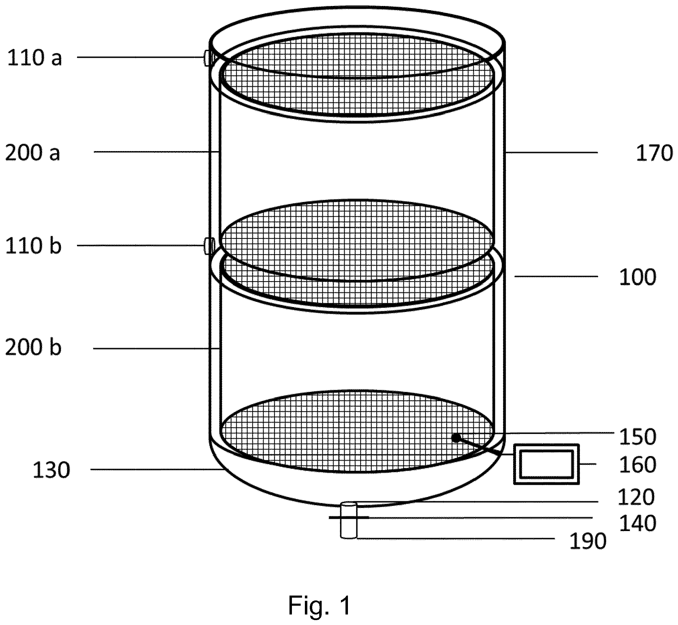

In Fig. 1 ist ein Desorptionsbehälter (100) dargestellt, der eine umlaufende Wand (170) und einen mit der umlaufenden Wand (170) abschließenden Boden (130) umfasst, zwei Dampfeingänge (110a, 110b) und einen am Boden des Desorptionsbehälters (130) angeordneten Dampfausgang (120) aufweist. Am Dampfausgang ist ein Ventil (140) angeordnet. Dieses Ventil (140) ist eingerichtet, die stromabwärts an den Dampfausgang (120) anschließende Ausleitung (190) zu öffnen oder zu verschließen. In dem Desorptionsbehälter (100) sind zwei entnehmbare Adsorbentienbehälter (200a, 200b) übereinander angeordnet. Die Dampfeingänge (110a, 100b) sind oberhalb der entnehmbaren Adsorbentienbehälter (200a, 200b) angeordnet. Zwischen dem Dampfausgang (120) und dem unteren Adsorbentienbehälter (200b) ist unmittelbar unterhalb des unteren Adsorbentienbehälters (200b) ein Temperatursensor (150) angeordnet. Dieser ist mit einer Steuereinrichtung (160) verbunden.

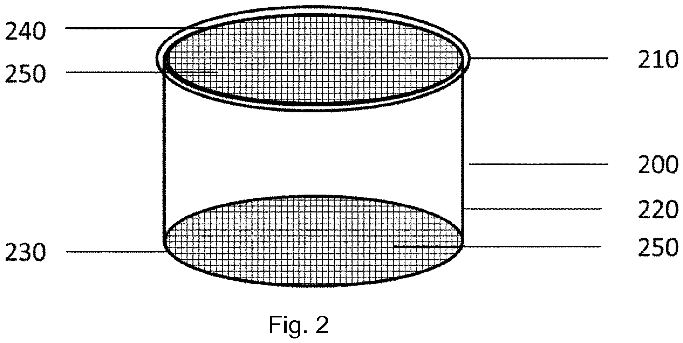

In Fig. 2 ist ein Adsorbentienbehälter (200) dargestellt. Dieser weist eine umlaufende Wand (220), damit abschließend einen Boden (230) und einen Deckel (240) auf. Der Boden (230) und der Deckel (240) des Adsorbentienbehälters sind mit einem Filtergewebe (250) ausgestaltet. Am Deckel (240) des Adsorbentienbehälters (200) ist eine Dichtung (210) angeordnet, die dazu eingerichtet ist, den Zwischenraum zwischen der Wand des Desorptionsbehälters (170) und des Adsorbentienbehälters (220) gasdicht zu verschließen, wenn der Adsorbentienbehälter in den Desorptionsbehälter eingeführt ist.

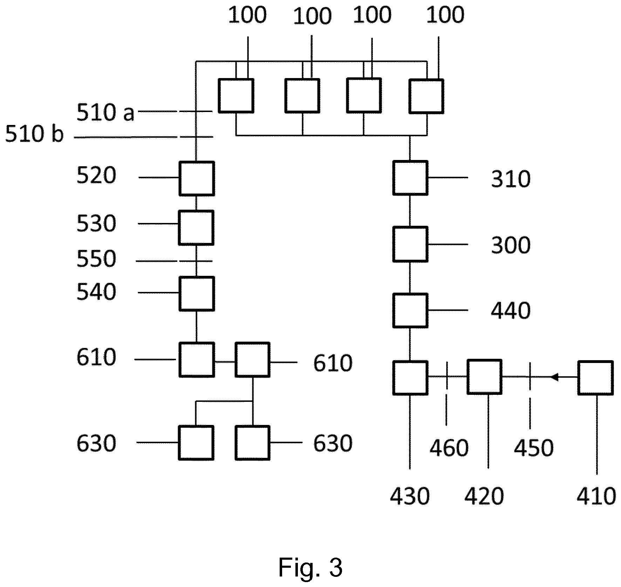

In Fig. 3 ist die Rückgewinnungsanlage schematisch dargestellt. In den Desorptionsbehältern (100) findet der Sterilisations- und Desorptionsvorgang statt. Hierzu benötigter trockener Reinstdampf wird im Dampferzeuger (300) mit nachgeschalteter Trocknungseinrichtung (310) bereitgestellt. Die Trocknungseinrichtung (310) kann eine Tröpfchenabscheidekolonne sein. Der Reinstdampf wird aus entsalztem Wasser hergestellt. Das entsalzte Wasser wird in einer dem Dampferzeuger (300) vorgeschalteten Reinigungseinrichtung bereitgestellt, welche dazu eingerichtet ist, Wasser aus einer Stadtwassereinspeisung (410) in einem ersten Schritt mittels einer Enthärtungsanlage (420) und in einem zweiten Schritt mittels eines Umkehrosmosemoduls (430) aufzureinigen. Zwischen Stadtwassereinspeisung (410) und Enthärtungsanlage (420) ist ein Vorfilter (450) angeordnet. Zwischen Enthärtungsanlage (420) und Umkehrosmosemodul (430) ist ein Feinfilter (460) angeordnet. Dem Umkehrosmosemodul (430) nachgeschaltet ist ein Wasserspeicher (440), der dazu eingerichtet ist, das aufgereinigte Stadtwasser für die Verwendung im Dampferzeuger (300) zu speichern. Die Desorptionsbehälter (100) sind zwischen dem Dampferzeuger (300) und einem Kondensatbereich angeordnet. Der Kondensatbereich ist dazu eingerichtet, das den Desorptionsbehälter (100) im Desorptionsschritt verlassende Gasgemisch zur Bildung eines Kondensats zu kühlen. Der Kondensatbereich umfasst einen Vorkühler (520), einen Zwischenkühler (530) sowie einen Nachkühler (540). Dem Vorkühler (520) vorgeschaltet sind ein Vorfilter (510a) und ein Nachfilter (510b). Zwischen Zwischenkühler (530) und Nachkühler (540) ist ein Feinfilter (550) angeordnet.

An den Kondensatbereich schließt sich ein Auffangbereich an. Ein oder mehrere miteinander verbundene Kondensatauffangbehälter (610) sind dem Nachkühler (540) nachgeschaltet und sind dazu eingerichtet, das den Nachkühler (540) passierende Kondensat aufzufangen und in seine Phasen aufzutrennen.

An den Kondensatauffangbehälter (610) schließen sich ein oder mehrere Pufferbehälter (630) an, die dazu eingerichtet sind, das in seine Phasen getrennte Kondensat aufzunehmen.

### Beschreibung beispielhafter Ausführungsformen

### Beispiel 1

Die Einbringung des Heißdampfes ist in zwei Phasen unterteilt. In der ersten Phase strömt der Heißdampf bei geschlossenen Ausgangsventil von dem Dampferzeuger in den Desorptionsbehälter bis ein Druck von größer/gleich 0,2 MPa erreicht wird. Dieser Druck und die damit korrespondierende Temperatur werden über einen Zeitraum von 30 min gehalten. In dieser Zeit herrschen autoklavenähnliche Verhältnisse im Desorptionsbehälter, die eine vollständige Inaktivierung aller im Sorbat befindlichen Pathogene (Bakterien, Mikroplasmen, Pilze, Viren, Viroide, Prionen, und/ oder Parasiten). gewährleisten. Nach dieser Inaktivierungsphase schließt sich die Desorptionsphase über einen Zeitraum von 120 min an, bei der bei geöffneten Ausgangsventil eine stetige Heißdampfströmung durch den Desorptionsbehälter eine Aufnahme und Abtransport der aus dem Sorbat ausgetriebenen halogenierten Kohlenwasserstoffe ermöglicht. Dieses Kondensat-Dampf-Gemisch wird über eine Sammelleitung mittels eines spülbaren Vorfilters (Edelstahlfilter, 25 Mikrometer) und einem spülbaren Nachfilter (Edelstahl, 5 Mikrometer) von den mitgeführten Verunreinigungen befreit und durch drei nacheinander geschaltete Kühler auf eine Temperatur von unter 30 Grad gebracht. Nach dem letzten Kühler wird das Kondensat-Wasser-Gemisch in einen Kondensatauffangbehälter überführt.

### Beispiel 2

**Tabelle 1: Übersicht über die Prozessdaten verschiedener Sterilisationszyklen.**

| **Versuch** | **Eingabe T/°C (+15 K)** | **Tₘᵢₙ/°C** | **Tₘₐₓ/°C** | **Druck/ bar** | **Zeit / min** | **Keime** |
|---|---|---|---|---|---|---|
| 1.1 | 121,11 (+15 K) | 80,6 | 127,56 | 2,4 | 28:46 min | An allen Positionen Keime |
| 1.2 | 121,11 (+15 K) | 116,62 | 130,93 | 2,5 | 28:50 min | An einer Position Keime |
| 1.3 | 121,11 (+15 K) | 124,59 | 141,44 | 2,4 | 28:10min | An allen Position keimfrei |
| 1.4 | 121,11 (+15 K) | 125,01 | 136,55 | 2,35 | 28:27 min | An 2 Positionen Keime |
| 2.1 | 121,11 (+15 K) | 126,30 | 134,57 | 2,44 | 32:53 min | An allen Positionen keimfrei |
| 2.2 | 121,11 (+15 K) | 100,40 | 130,07 | N/A | 34:49 min | An 2 Positionen Keime |
| 2.3 | 121,11(+15 K) | 64,03 | 128,16 | 2,48 | 30:15 min | An 6 Positionen Keime |
| 2.4 | 121,11 (+15 K) | 121,68 | 129,29 | 2,5 | 28:27 | An allen Positionen keimfrei |
| 2.5 | 121,11 (+15K) | 127,25 | 131,34 | 2,5 | 30:22 | An allen Positionen keimfrei |
| | | | | | | |
| 3.1 | 121,1 (+25K) | 134,08 | 144,57 | 3,0 | 33:24 | Keine Angaben |
| 3.2 | 121,1 (+25K) | 132,15 | 146,46 | 3,0 | 37:31 | Keine Angaben |
| 3.3 | 121,1(+25K) | 131,0 | 143,96 | 3,0 | 36:44 | Keine Angaben |
| 3.4 | 121,11 (+25K) | 134,89 | 138,08 | 3,0 | 29:03 | Keine Angaben |

### Bezugszeichenliste

- 100: Desorptionsbehälter
- 110: Dampfeingang
- 110 a: erster Dampfeingang
- 110 b: zweiter Dampfeingang
- 120: Dampfausgang
- 130: Boden des Desorptionsbehälters
- 140: Ventil
- 150: Temperatursensor
- 160: Steuereinrichtung
- 170: Wand des Desorptionsbehälters
- 190: Ausleitung
- 200: Adsorbentienbehälter
- 210: Dichtung
- 220: Wand des Adsorbentienbehälters
- 230: Boden des Adsorbentienbehälters
- 240: Deckel des Adsorbentienbehälters
- 250: Filtergewebe
- 300: Dampferzeuger
- 310: Trocknungseinrichtung
- 410: Stadtwassereinspeisung
- 420: Enthärtungsanlage
- 430: Umkehrosmosemodul
- 440: Wasserspeicher
- 450: Vorfilter
- 460: Feinfilter
- 510: Filter
- 510 a: Vorfilter
- 510 b: Nachfilter
- 520: Vorkühler
- 530: Zwischenkühler
- 540: Nachkühler
- 550: Feinfilter
- 610: Kondensatauffangbehälter
- 620: Drucklufterzeuger
- 630: Pufferbehälter

## Patentansprüche

1. Verfahren zur Rückgewinnung von fluorhaltigen Inhalationsanästhetika,
wobei in einem Desorptionsschritt ein fluorhaltige Inhalationsanästhetika umfassendes Adsorbens von Wasserdampf durchströmt wird, wodurch ein fluorhaltige Inhalationsanästhetika enthaltender sekundärer Volumenstrom entsteht,
und wobei der sekundäre Volumenstrom durch Kühlung in ein fluorhaltige Inhalationsanästhetika und Wasser enthaltendes Kondensat überführt wird, aus welchem die fluorhaltigen Inhalationsanästhetika abgetrennt werden,
**dadurch gekennzeichnet, dass**
in einem dem Desorptionsschritt vorangehenden Sterilisationsschritt das adsorbierte fluorhaltige Inhalationsanästhetika umfassende Adsorbens
- für 10 bis 60 min,
- bei einer Temperatur von 121 bis 150°C, und
- bei einem Druck von 0,15 MPa bis 0,4 MPa, insbesondere von 0,15 bis 0,3 MPa in Kontakt mit Wasserdampf gebracht wird,
- wobei der Sterilisationsschritt und der Desorptionsschritt in derselben Anlage direkt aufeinanderfolgend durchgeführt werden,
- wobei der Sterilisationsschritt und der Desorptionsschritt in einem Desorptionsbehälter (100) durchgeführt werden,
- und wobei der Desorptionsbehälter (100) einen Dampfeingang (110) und einen Dampfausgang (120) aufweist, und das Adsorbens zwischen Dampfeingang (110) und Dampfausgang (120) so in dem Desorptionsbehälter (100) angeordnet wird, dass in den Desorptionsbehälter (100) durch den Dampfeingang (110) eintretender Dampf das Adsorbens durchströmen muss, bevor es den Desorptionsbehälter (100) durch den Dampfausgang (120) verlässt,
- und wobei der Desorptionsbehälter (100) ein Ventil (140) aufweist, welches dem Dampfausgang (120) nachgeschaltet ist, wobei das Ventil (140) während des Sterilisationsschrittes geschlossen ist und während des Desorptionsschrittes geöffnet ist,
- und wobei die in dem Desorptionsbehälter (100) herrschende Temperatur durch einen Temperatursensor (150) gemessen wird, und wobei der Temperatursensor (150) sich unterhalb eines in den Desorptionsbehälter (100) eingebrachten Adsorbentienbehälters (200) befindet,
und wobei das die adsorbierten fluorhaltigen Inhalationsanästhetika umfassende Adsorbens durch Filterung von Atemluft aus der Behandlung von Patienten erhalten wurde.

2. Das Verfahren gemäß einem der vorstehenden Ansprüche, wobei der Sterilisationsschritt
- für 20 bis 40 min, insbesondere für ungefähr 30 min
- bei einer Temperatur von 135 bis 145°C, und
- bei einem Druck von 0,24 bis 0,26 MPa
durchgeführt wird.

3. Das Verfahren gemäß einem der vorstehenden Ansprüche, wobei der Temperatursensor (150) sich zwischen dem Dampfausgang (120) und dem in den Desorptionsbehälter (100) eingebrachten Adsorbentienbehälter (200), insbesondere unmittelbar unterhalb des in den Desorptionsbehälter (100) eingebrachten Adsorbentienbehälters (200), befindet.

4. Das Verfahren gemäß einem der vorstehenden Ansprüche, wobei der Desorptionsbehälter (100) zwei übereinander angeordnete Adsorbentienbehälter (200a, 200b) umfasst, und die Adsorbentienbehälter (200a, 200b) durch zwei jeweils über den Adsorbentienbehältern (200a, 200b) angeordnete Dampfeingänge (110a, 110b) mit Dampf beaufschlagt werden können.

5. Das Verfahren gemäß einem der Ansprüche 4 oder 5, wobei der Adsorbentienbehälter (200) einen Boden (230) und/oder einen Deckel (240) aufweist, wobei der Boden (230) und/oder der Deckel (240) ein gasdurchlässiges Filtergewebe (250) umfassen oder daraus bestehen.

6. Eine Vorrichtung zur Durchführung eines Verfahrens gemäß einem der vorstehenden Ansprüche, umfassend
- einen druckstabilen Desorptionsbehälter (100),
- einen zum Einlass von Wasserdampf in den Desorptionsbehälter (100) eingerichteten Dampfeingang (110),
- einen Dampferzeuger (300), der zur Erzeugung von Wasserdampf eingerichtet ist, wobei der Dampferzeuger (300) mit dem Dampfeingang (110) in fluidischer Kommunikation steht,
- einen zum Abführen von Wasserdampf aus dem Desorptionsbehälter (100) eingerichteten Dampfausgang (120) mit einer in Richtung des Dampfaustritts hinter dem Dampfausgang (120) angeordneten Ausleitung (190);
- ein Ventil (140), durch welches die Ausleitung (190) verschließbar ist,
- einen Raum, der zur Aufnahme eines Schüttguts ausgebildet ist, wobei das Schüttgut ein fluorhaltige Inhalationsanästhetika umfassendes Adsorbens ist,
- wobei der Desorptionsbehälter (100) einen zur Aufnahme des Schüttguts vorgesehenen Adsorbentienbehälter (200), welcher den Raum zur Aufnahme des Schüttguts ausbildet, und einen Temperatursensor (150), welcher auf der dem Dampfausgang (120) zugewandten Seite des Adsorbentienbehälters (200) angeordnet ist, umfasst,
- einen mit dem Desorptionsbehälter (100) verbundenen Kondensatbereich, der dazu eingerichtet ist, einen fluorhaltige Inhalationsanästhetika und Wasserdampf enthaltenden sekundären Volumenstrom zu kühlen, der in einem Desorptionsschritt durch Durchströmen des fluorhaltige Inhalationsanästhetika umfassenden Adsorbens mit dem Wasserdampf entsteht, und aus dem sekundären Volumenstrom ein Kondensat zu erzeugen, und wobei
- der Desorptionsbehälter (100) bis 0,4 MPa druckstabil ist.

7. Die Vorrichtung gemäß Anspruch 7, wobei der Temperatursensor (150) mit einer Steuereinrichtung (160) verbunden ist, welche dazu ausgebildet ist, das Ventil (140) nach Erreichen einer Zieltemperatur und/oder nach Ablauf einer vorgewählten Zeitspanne von 10 bis 60 min, während derer die Zieltemperatur gehalten wird, zu öffnen.

8. Die Vorrichtung gemäß einem der Ansprüche 7 bis 9, wobei der Desorptionsbehälter zur Aufnahme zweier übereinander angeordneter Adsorbentienbehälter (200a, 200b) eingerichtet, und mit zwei jeweils oberhalb der Adsorbentienbehälter (200a, 200b) angeordneten, Dampfeingängen (110a, 110b) ausgestaltet ist.

## Claims

1. Process for the recovery of fluorine-containing inhalation anaesthetics,
wherein in a desorption step an adsorbent comprising fluorine-containing inhalation anaesthetics is flowed through by water vapour, resulting in a secondary volume flow comprising fluorine-containing inhalation anaesthetics,
and wherein the secondary volume flow is converted by cooling into a condensate containing fluorine-containing inhalation anaesthetics and water, from which the fluorine-containing inhalation anaesthetics are separated,
**characterised in that**
in a sterilisation step preceding the desorption step, the adsorbent comprising adsorbed fluorine-containing inhalation anaesthetics is brought into contact with water vapour
- for 10 to 60 min,
- at a temperature from 121 to 150°C, and
- at a pressure of from 0.15 MPa to 0.4 MPa, in particular from 0.15 to 0.3 MPa,
- wherein the sterilisation step and the desorption step are carried out in direct sequence in the same installation,
- wherein the sterilisation step and the desorption step are carried out in a desorption vessel (100),
- and wherein the desorption vessel (100) comprises a steam inlet (110) and a steam outlet (120), and the adsorbent is positioned between the steam inlet (110) and the steam outlet (120) in the desorption vessel (100) such that steam entering the desorption vessel (100) through the steam inlet (110) must pass through the adsorbent before leaving the desorption vessel (100) through the steam outlet (120),
- and wherein the desorption vessel (100) comprises a valve (140) connected downstream of the steam outlet (120), the valve (140) being closed during the sterilisation step and open during the desorption step,
- and wherein the temperature prevailing in the desorption vessel (100) is measured by a temperature sensor (150), and wherein the temperature sensor (150) is located below an adsorbent container (200) placed in the desorption vessel (100), and
wherein the adsorbent comprising the adsorbed fluorine-containing inhalation anaesthetics has been obtained by filtering breathing air from the treatment of patients.

2. The process according to any one of the preceding claims, wherein the sterilisation step is carried out
- for 20 to 40 min, in particular for about 30 min
- at a temperature of 135 to 145°C, and
- at a pressure of 0.24 to 0.26 MPa.

3. The method according to any one of the preceding claims, wherein the temperature sensor (150) is located between the steam outlet (120) and the adsorbent container (200) placed in the desorption container (100), in particular immediately below the adsorbent container (200) placed in the desorption container (100).

4. The method according to any one of the preceding claims, wherein the desorption vessel (100) comprises two adsorbent vessels (200a, 200b) arranged one above the other, and the adsorbent vessels (200a, 200b) can be supplied with steam through two steam inlets (110a, 110b) arranged above the adsorbent vessels (200a, 200b), respectively.

5. The method according to any one of claims 4 or 5, wherein the adsorbent container (200) comprises a bottom (230) and/or a lid (240), the bottom (230) and/or the lid (240) comprising or consisting of a gas-permeable filter fabric (250).

6. An apparatus for carrying out a process according to any one of the preceding claims, comprising
- a pressure-stable desorption vessel (100),
- a steam inlet (110) configured to admit steam into the desorption vessel (100),
- a steam generator (300) adapted to generate steam, the steam generator (300) being in fluid communication with the steam inlet (110),
- a steam outlet (120) configured for discharging steam from the desorption vessel (100), with an outlet (190) arranged downstream of the steam outlet (120) in the direction of the steam discharge;
- a valve (140) by which the outlet (190) can be closed,
- a space adapted to receive a bulk material, the bulk material being an adsorbent comprising fluorine-containing inhalation anaesthetics,
- wherein the desorption container (100) comprises an adsorbent container (200) for receiving the bulk material, the adsorbent container (200) forming the space for receiving the bulk material, and a temperature sensor (150) arranged on the side of the adsorbent container (200) facing the vapour outlet (120),
- a condensate section connected to the desorption container (100) and configured to cool a secondary volume flow containing fluorine-containing inhalation anaesthetics and water vapour, which is produced in a desorption step by flowing the water vapour through the adsorbent comprising fluorine-containing inhalation anaesthetics, and to produce a condensate from the secondary volume flow, and
- wherein the desorption vessel (100) is pressure stable up to 0.4 MPa.

7. The apparatus according to claim 7, wherein the temperature sensor (150) is connected to a control device (160) configured to open the valve (140) after a target temperature has been reached and/or after a preselected time period of 10 to 60 min has elapsed during which the target temperature is maintained.

8. The apparatus according to any one of claims 7 to 9, wherein the desorption vessel is configured to receive two adsorbent vessels (200a, 200b) arranged one above the other, and is configured with two steam inlets (110a, 110b) arranged respectively above the adsorbent vessels (200a, 200b).

## Revendications

1. Procédé de récupération d'anesthésiques fluorés par inhalation,
dans lequel un adsorbant renfermant des anesthésiques fluorés par inhalation est traversé par de la vapeur d'eau au cours d'une étape de désorption, ce qui fait apparaître un flux volumique secondaire contenant des anesthésiques fluorés par inhalation,
et dans lequel le flux volumique secondaire est transformé par refroidissement en un condensat contenant des anesthésiques fluorés par inhalation et de l'eau, à partir duquel des anesthésiques fluorés par inhalation sont séparés, **caractérisé en ce que**
l'adsorbant renfermant les anesthésiques fluorés par inhalation adsorbés est mis en contact avec de la vapeur d'eau
- pendant 10 à 60 min.,
- à une température de 121 à 150 °C, et
- à une pression de 0,15 MPa à 0,4 MPa, en particulier de 0,15 à 0,3 MPa dans une étape de stérilisation précédant l'étape de désorption,
- dans lequel l'étape de stérilisation et l'étape de désorption sont exécutées directement l'une après l'autre dans la même installation,
- dans lequel l'étape de stérilisation et l'étape de désorption sont exécutées dans un récipient de désorption (100),
- et dans lequel le récipient de désorption (100) présente une entrée de vapeur (110) et une sortie de vapeur (120), et l'adsorbant est disposé entre l'entrée de vapeur (110) et la sortie de vapeur (120) dans le récipient de désorption (100), de sorte que la vapeur ayant pénétré dans le récipient de désorption (100) par l'entrée de vapeur (110) doive traverser l'adsorbant, avant que celui-ci ne quitte le récipient de désorption (100) par la sortie de vapeur (120),
- et dans lequel le récipient de désorption (100) présente une soupape à vapeur (140), qui est montée en aval de la sortie de vapeur (120), dans lequel la soupape à vapeur (140) est fermée durant l'étape de stérilisation et ouverte durant l'étape de désorption,
- et dans lequel la température régnant dans le récipient de désorption (100) est mesurée par un capteur de température (150), et dans lequel le capteur de température (150) se trouve en dessous d'un récipient d'adsorbants (200) introduit dans le récipient de désorption (100) et
dans lequel l'adsorbant renfermant les anesthésiques fluorés par inhalation adsorbés a été conservé par filtrage de l'air respiratoire issu du traitement de patients

2. Le procédé selon l'une des revendications précédentes, dans lequel l'étape de stérilisation est exécutée
- pendant 20 à 40 min., en particulier pendant environ 30 min.,
- à une température de 135 à 145 °C, et
- à une pression de 0,24 MPa à 0,26 MPa.

3. Le procédé selon l'une des revendications précédentes, dans lequel le capteur de température (150) se trouve entre la sortie de vapeur (120) et le récipient d'adsorbants (200) introduit dans le récipient de désorption (100), notamment juste en dessous du récipient d'adsorbants (200) introduit dans le récipient de désorption (100).

4. Le procédé selon l'une des revendications précédentes, dans lequel le récipient de désorption (100) comprend deux récipients d'adsorbants (200a, 200b) disposés l'un au-dessus de l'autre et les récipients d'adsorbants (200a, 200b) peuvent être alimentés en vapeur par les deux entrées de vapeur (110a, 110b) disposées respectivement au-dessus des récipients d'adsorbants (200a, 200b).

5. Le procédé selon l'une des revendications 4 ou 5, dans lequel le récipient d'adsorbants (200) présente un fond (230) et / ou un couvercle (240), dans lequel le fond (230) et / ou le couvercle (240) comprennent un tissu filtrant (250) perméable aux gaz ou en sont constitués.

6. Un dispositif pour exécuter un procédé selon l'une des revendications précédentes, comprenant
- un récipient de désorption (100) résistant à la pression,
- une entrée de vapeur (110) ajustée pour l'admission de vapeur d'eau dans le récipient de désorption (100),
- un générateur de vapeur (300), qui est ajusté pour produire de la vapeur d'eau, dans lequel le générateur de vapeur (300) est en communication fluidique avec l'entrée de vapeur (110),
- une sortie de vapeur (120) ajustée pour l'évacuation de vapeur d'eau du récipient de désorption (100) par un conduit d'évacuation (190) disposé dans la direction du dégagement de vapeur derrière la sortie de vapeur (120),
- une soupape de vapeur(140), au moyen de laquelle le conduit d'évacuation (190) peut être fermé,
- un espace, qui est conçu pour recevoir un produit en vrac, dans lequel le produit en vrac est un adsorbant renfermant des anesthésiques fluorés par inhalation,
- dans lequel le récipient de désorption (100) comprend un récipient d'adsorbants (200) prévu pour recevoir le produit en vrac, lequel forme l'espace destiné à recevoir le produit en vrac, et un capteur de température (150), lequel est disposé du côté tourné vers la sortie de vapeur (120) du récipient d'adsorbants (200),
- une zone de condensat reliée au récipient de désorption (100), qui est ajustée afin de refroidir un flux volumique secondaire contenant des anesthésiques fluorés par inhalation et de la vapeur d'eau, qui apparaît moyennant le passage de la vapeur d'eau à travers l'adsorbant renfermant des anesthésiques fluorés par inhalation au cours d'une étape de désorption, et afin de générer un condensat à partir du flux volumique secondaire, et dans lequel le récipient de désorption (100) est résistant à la pression jusqu'à 0,4 MPa

7. Le dispositif selon la revendication 7, dans lequel le capteur de température (150) est relié à un système de commande (160), lequel est conçu de manière à ouvrir la soupape (140) après avoir atteint une température cible et / ou après l'écoulement d'un intervalle de temps présélectionné de 10 à 60 min., durant lequel la température cible est maintenue.

8. Le dispositif selon l'une des revendications 7 à 9, dans lequel le récipient de désorption est ajusté pour recevoir deux récipients d'adsorbants (200a, 200b) disposés l'un au-dessus de l'autre et est équipé de deux entrées de vapeur (110a, 110b) disposées respectivement au-dessus des récipients d'adsorbants (200a, 200b).
